# EUROPEAN PATENT SPECIFICATION

(11) **EP 0 296 564 B1**
(45) Date of publication and mention of the grant of the patent: **28.12.1994**
(21) Application number: 88109936.0
(22) Date of filing: 22.06.1988
(51) Int. Cl.: C07D 307/92, C07C 69/63, C07D 303/16, C07D 311/92

(54) **Method for preparing dodecahydro-3a,6,6,9a-tetramethylnaphtho[2,1-b] furan and novel haloethyl decalin derivatives**
Verfahren zur Herstellung von Dodecahydro-3a,6,6,9a-tetramethylnaphtho[2,1b]furan und von Haloethyldecalin-Derivaten
Procédé pour la préparation de dodécahydro-3a,6,6,9a-tétraméthylnaphtho[2,1b]furanne et de dérivés d'haloéthyl-décaline

(30) Priority: 23.06.1987 US 65426
(43) Date of publication of application: 28.12.1988
(60) Divisional application: 92110558.1
(73) Proprietor: GIVAUDAN-ROURE (INTERNATIONAL) S.A., CH-1214 Vernier (CH)
(72) Inventor: Christenson, Philip A., Midland Park New Jersey 07432 (US)
(74) Representative: VOSSIUS & PARTNER

(56) References cited:
- EP-A- 0 170 995
- DE-A- 3 610 063
- PATENT ABSTRACTS OF JAPAN vol. 9, n0. 196(C-297) (1919), 13 August 1985;
- HELVETICA CHEMICA ACTA vol. XXXIII, Fasc. V no. 160-161, 1950, pages 1251-1260 ; M Stoll et al.: "Odeur et Constitution III. Les substances bicyclohomofarnèsiques" page1252, schemeI,II,III
- JOURNAL CHEMICAL RESEARCH (S) 1981, pages 236-237; N Bensadoun et al.: "Stereoelectronic Control in the beta-Fragmentation reactions of Alkoxyl Radicals. An Empirical Predictive Rule"
- TETRAHEDRON LETTERS vol. 26, no. 45, November 1985, pages 5717-5720, Oxford, New York, Frankfurt; J G Urones et al.: "Functionalization at C-12 inlabdanic ditteapenes: Synthesis of the Natural Diterpenic lactones isolated from Cistrus Ladaniferus L ".
- JOURNAL OF THE CHEMICAL SOCIETY no. 13, 6 July 1977, pages 478-479, London, GB; Z Majerski et al.: "Hypoiodite Thermolysis-Cyclization Reaction. Convenient Synthesis of 4-Homoprotoadamanten-4-one from 3-Homoadamantanol".
- HOUBEN-WEYL: METHODEN DER ORGANISCHEN CHEMIE vol. IV/b, 1975, pages 939-952, Stuttgart.
- SYNTHESIS, September 1984, pages 704-726, Stuttgart; A Varvoglis: "Polyvalent lodine Compounds in Organic Synthesis"
- JCS 1960, 4613-4627, Aust J Chem 1972, 25, 365-374

## Description

The compound dodecahydro-3a,6,6,9a-tetramethylnaphtho [2,1-b]furan (hereinafter Naphthofuran II) is a synthetic congener of Ambergris, a rare perfumery composition of natural origin having a sweet, woody, amber bouquet. Naphthofuran II has been used in perfume compositions and in cleaning formulations, such as fragrances for toiletries and household products, where a persistent amber effect is desired. Naphthofuran II is also a component of tincture of Ambergris and synthetic Naphthofuran II has been used in artificial Ambergris formulations.

Naphthofuran II may be produced synthetically from 3-ethenyldecahydro -2-hydroxy-α-2,5,5,8a-pentamethyl-1-naphthalenepropanol, commonly known as sclareol. One method for this conversion is a two stage oxidation followed by a hydride reduction and cyclization. See, for example, U.S. Patent No. 3,050,532; U.S. Patent No. 3,029,255; and Aust. J. Chem., 1971, 24, 591. Another method involves oxidation of sclareol to sclareol oxide followed by a mutli-step synthesis to produce Napthofuran II as a mixture of stereoisomers. See, for example, Helv. Chim. Acta, 1942, 25 621; J. Chem. Soc., 1960, 4613; Helv. Chim. Acta, 1950, 33, 1251; U.S. Patent 3,029,255, 1962; Helv. Chim. Acta, 1950, 33 1308; J. Am. Chem. Soc., 1963, 85 3979); and Helv. Chim. Acta, 1951, 34 1664.

These methods, however, lack simplicity and are based upon multiple synthetic reactions. Moreover, they produce undesirable side reactions and do not generate high yields of Naphthofuran II. Accordingly, it is an object of the invention to develop a simple method for the production of Naphthofuran II from sclareol. Another object is the production of a high yield of Naphthofuran II without the complications caused by side reactions.

These and other objects are achieved by the invention which is directed to a method for the production of Naphthofuran (II) from sclareol. In this method, an alkoxy-radical fragmentation reaction is used to convert sclareol derivatives to a 9-haloethyl decalin derivative of the formula I 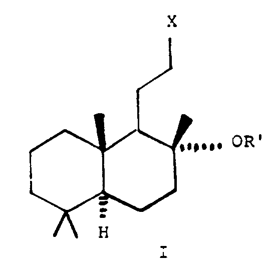
wherein X is iodo, bromo or chloro and R' is an alkanoyl group of 2 to 5 carbons. Basic hydrolysis of this 9-haloethyl decalin derivative produces Naphthofuran of the formula II. 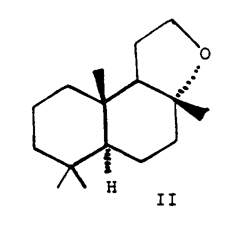
According to the invention sclareol oxide of the formula IV 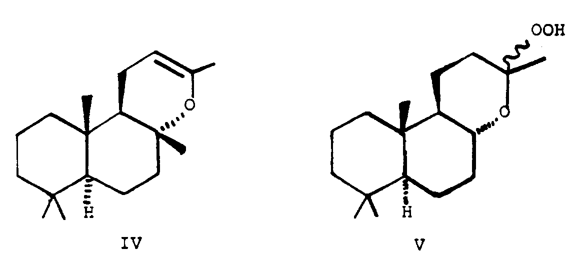
is treated with hydrogen peroxide in the presence of an acid catalyst to produce a peroxide intermediate V which is then treated with a metallic halide reducing agent to produce the decalin derivative of formula I.

The invention is directed as well to the novel 9-haloethyl decalin derivatives of formula I and a sclareol oxide-13-hydroperoxide intermediate V.

The process of the present invention produces a high yield of dodecahydro-3a,6,6,9a-tetramethylnaphtho[2,1-b]furan (Napthofuran II) from sclareol derivatives through a sequence of steps which employ an alkoxy-radical fragmentation reaction and a hydrolysis. It avoids production of side products that could alter the fragrance qualities of Napthofuran II.

The alkoxy-radical reaction employed in the process of the present invention, treatment of sclareol oxide with a source for hydrogen peroxide such as hydrogen peroxide itself, or a hydrogen peroxide generating system in an inert solvent in the presence of an acid such as acetic acid gives a hydroperoxide intermediate. An example of a hydrogen peroxide generating system which may be useful comprises a mixture of barium peroxide and a protic acid such as acetic acid or carbonic acid. Other protonic or Bronsted acids can also be used. Preferred acids include lower alkyl carboxylic acids, chloro, dichloro and trifluoroacetic acid, formic acid, phosphoric acid and buffers derived from phosphoric acid. Acetic acid is especially preferred. The amount of acid used may be from about 10 to about 5000 mole percent with about 100 to about 4000 mole percent being preferred and about 3500 mole percent being especially preferred, the mole percentage being measured relative to the amount of sclareol oxide present. Solvents such as dimethoxyethane, dioxane, ethyl ether, t-butanol, ethyl acetate, dichloromethane, tetrahydrofuran, dichloroethane, water, t-butyl methyl ether, toluene and hexane may also be used. t-Butyl methyl ether, t-butanol and tetrahydrofuran are preferred, the latter being especially preferred. A useful temperature range is between about 0° and about 50°C with about 10° to about 40° being preferred and about 20° to about 30°C being the most preferred. The peroxide treatment can take from about 0.5 to about 10 hours, about 0.5 to about 6 hours being preferred and about 3 to 4 hours being especially preferred.

The hydroperoxide intermediate can be fragmented to provide the decalin derivative of formula I wherein X is Cl, Br or I upon treatment with a metal halide reducing agent such as ferrous halide and a catalytic amount of a cupric halide, the halide being Cl, Br or I. Other metal halide reducing agents may also be used in this reaction, including halide salts copper (I), chromium (II), titanium (III), vanadium (II) or (III), cobalt (II) or manganese (II). Useful solvents include alkanols such as methanol, ethanol, isopropanol and t-butanol as well as water, tetrahydrofuran,dioxane, ether and mixtures thereof. The alcohols, tetrahydrofuran and water are preferred, with methanol being especially preferred. The reaction may be performed in the temperature range of about 0° to about 50°C preferrably about 10° to about 35°C, most preferrably 20° to about 30°C. The time for the reaction is 0.25 to about 2 hours with about 0.5 to about 2 hours being preferred and about 0.5 to 1 hour being especially preferred.

The haloethyl decalin derivatives of formula I produced in the foregoing fashion are converted to Naphthofuran II in high yield according to the process of the present invention by hydrolysis with a metal hydroxide in a solvent therefor. Alkali or alkaline earth metal hydroxides are preferred. Potassium hydroxide is the most preferred. A variety of protic or aprotic solvents mixed with water may be used, such as lower alkyl alcohols, ethyl ether, tetrahydrofuran, dioxane, dimethoxyethane, dimethylsulfoxide, ethylene glycol, dimethylformamide, N-methylpyrrolidone or acetonitrile. The preferred solvents are a mixture of water and alcohols or diols, such as, methanol, ethanol, propanol, isopropanol, n-butanol, t-butanol, ethylene glycol. The most preferred solvent is a mixture of water and isopropanol. The reaction may be carried out in the temperature range of about 25° to about 150°C. The preferred temperature range is about 50° to about 100°C. The most preferred temperature range is about 70° to about 85°C.

The process of the present invention may be applied to other labdane derivatives such as manool(VI), larixol (VII), torulosol (VIII) and cupressic acid (IX) to provide compounds of general formula X, wherein R and R' are as described in VI-VIII and X is halogen. 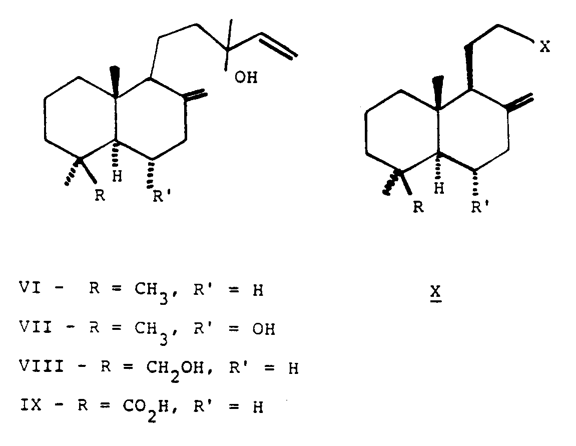
Compounds X may be converted into useful terpenoid derivatives.

Practice of other embodiments of the invention will be apparent from the foregoing discussion. The alkoxy-radical fragmentation can be applied pursuant to reaction procedures disclosed by Waegell et al., J. Chem. Res. S., 1981, 236. The following examples illustrate some further embodiments of the invention.

### Experimental Section

General. Benzene was dried over 4A molecular sieves prior to use. Sclareol, purchased from R.J. Reynolds Tobacco Co., was recrystallized from hexane prior to use. All other reagents and solvents were of reagent grade and were used as received.

IR spectra were obtained with a Perkin-Elmer 710B spectrophotometer. Routine ¹H-NMR spectra were recorded with a Varian Associates T-60A NMR instrument. The ¹H-NMR spectral data reported at 250 MHz were recorded on a Bruker WM250 NMR instrument and the ¹³C-NMR spectra were obtained on the same instrument at 62.5 MHz. Mass spectra were obtained with a Hewlett-Packard 5985 mass spectrophotometer. Column chromatography was performed with Merck 60 brand of silica gel. GLC analyses were obtained with a Hewlett-Packard Model 5840 or a Perkin-Elmer model 920 gas chromatograph, using either a 6 ft., 2-mm i.d. glass column packed with 3% nonpolar silicone on diatomaceous earth, 100-120 mesh or a 10 ft., 2 mm i.d. glass column packed with 2% polyethylene glycol on diatomaceous earth, 100-120 mesh. Where indicated, percentages refer to computer calculated peak areas without correction for response. GLC and mass spectral data were provided courtesy of the Fritzsche, Dodge and Olcott, Inc., Instrumental Laboratory.

Elemental microanalyses were performed by Childers Laboratories, Milford, N.J. or Schwarzkopf Microanalytical Laboratory, Woodside, N.Y. Melting points were determined with a Thomas Model 40 micro hot-stage apparatus and are uncorrected. Optical rotations were obtained in chloroform solution at ambient temperature unless otherwise noted using a Perkin-Elmer 241 polarimeter.

### EXAMPLE 1

### Decalin Derivative

Acetic acid (2 mL) was added to a mixture of sclareol oxide (5) (0.262g, 0.001 mol, which may be prepared as described by D.B. Bigley, N.A.J. Rogers and J.A. Barltrop in J Chem. Soc., 1960, 4613-4627), tetrahydrofuran (8 mL), and 30% hydrogen peroxide (6 mL). The mixture was stirred at 25°C for 4h. The mixture was poured onto water (10 mL) and extracted with hexane/ethyl acetate (9:1, 4 x 10 mL). The extracts were washed with water (2 x 5 mL), saturated sodium bicarbonate solution (2 x 10 mL) and dried (Na₂SO₄). Evaporation of solvents provided 0.327g of the hydroperoxide intermediate as a colorless solid. ¹H-NMR(60 MHz, CDCl₃) δ 0.080 (6H, s), 0.87 (3H, s) 1.38 (3H, s), 1.43 (3H, s), 0.8-2.2 (16H, m) 7.52 (1H, s).

A solution of the hydroperoxide intermediate (0.001 mol) in methanol (10 mL) was added dropwise over a 30 min period to a solution of ferrous chloride (0.398g, 0.002 mol) and cupric chloride (0.034g, 0.0002 mol) in methanol (6 mL) at 25°C. The mixture was stirred at 25°C for 15 min. The mixture was poured onto water (20 mL) and extracted with hexane/ethyl acetate (9:1, 4 x 15 ml). The extracts were washed with water (2 x 10 mL), saturated sodium bicarbonate solution (2 x 10 mL) and dried (Na₂SO₄). The solvents were evaporated and the residue chromatographed to provide 0.229g (73% yield) of [1R-[1α,2β,4aβ,8aα)]-decahydro-1-(2-chloroethyl)-2,5,5,8a - tetramethyl-2-naphthalenol acetate, the decalin derivative of formula 1 wherein X is Cl and R' is COCH₃. Recrystallization from hexane provided an analytical sample, mp 99-101.5°C, [α]D -15.76 (c, 1.76). ¹H- NMR (250 MHz, CDCl₃) δ 0.73 (3H, s), 0.78 (3H, s), 0.81 (3H, s), 1.43 (3H, s), 1.87 (3H, s), 0.7-2.05 (13H, m), 2.6-2.7 (1H, m), 3.36-3.59 (2H, m); ¹³C-NMR δ 15.56 (q), 18.28 (t), 19.87 (t), 20.39 (q), 21.37 (q), 22.65 (q), 29.88 (t), 33.07 (s), 33.22 (q), 38.87 (t), 39.09 (s), 39.68 (t), 41.78 (t), 45.76 (t), 55.59 (d), 56.93 (d), 87.00 (s), 169.30 (s); IR (CHCl₃) v ₘₐₓ 2930, 1725, 1460, 1440, 1390, 1370, 1255 cm⁻¹; MS, m/e 272, 256, 254, 241, 239, 137, 124, 109. Anal. Calcd for C₁₈H₃₁Cl0₂: C, 68.65; H, 9.92; Cl, 11.26. Found: C, 68.82; H, 10.08; Cl, 11.12.

### EXAMPLE 2

### Decalin Derivative

[1R-(1α,2β,4aβ,8aα)]-Decahydro-1-(2-bromoethyl)-2,5,5,8a -tetramethyl-2-naphthalenol acetate. The decalin derivative of forumla I wherein X is Br may be prepared by reaction of the hydro peroxide intermediate of Example 1 with ferrous bromide and cupric bromide in a manner similar to that described in Example 1. 20.72 (t), 22.69 (2q), 33.10 (s), 36.27 (s), 39.36 (t), 40.06 (t), 42.54 (t), 57.36 (d), 60.22 (d), 64.94 (t), 79.84 (s); IR (melt) v max 2930, 1480, 1460, 1390, 1375 cm⁻¹; MS, m/e 236, 221, 204, 137, 97.

### EXAMPLE 3

### Dodecahydro-3a,6,6,9a-tetramethylnaphtho[2,1-b]furan

A mixture of the decalin derivative of forumla I wherein X is Cl and R' is COCH₃ (0.124g, 0.36 mmol from Example 1) potassium hydroxide (0.118g) isopropanol (10 mL) and water (1.5 mL) was heated at reflux for 18h. The mixture was cooled and concentrated under reduced pressure. The residue was added to water and extracted with hexane (4 x 20 mL). The extracts were washed with water (15 mL), brine (15 mL) and dried (Na₂SO₄). The solvents were evaporated and the residue chromatographed (eluant;hexane:ethyl acetate; 20:1). Kugelrohr distillation (bath 120°C, 1 mm) gave 0.077g (92% yield, 85% pure according to GLC analysis) of Napthofuran II.

## Claims (Claims for the following Contracting State(s): AT, BE, CH, DE, FR, GB, GR, IT, LI, LU, NL, SE)

1. A method for the preparation of a decalin derivative of the formula 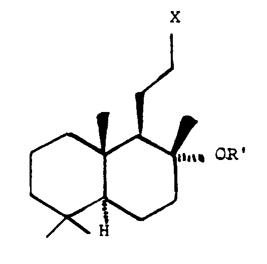 wherein X is chloro, bromo or iodo and R' is an alkanoyl group of 2 to 5 carbons, which comprises
treating sclareol oxide of the formula 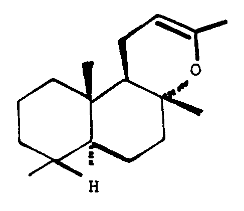 with hydrogen peroxide or a hydrogen peroxide generating system in the presence of an acid catalyst, and
treating the produced hydroperoxide intermediate with a metallic halide reducing agent, the halide being chloride, bromide or iodide.

2. A method for the production of dodecahydro-3a,6,6,9a-tetramethyl-naphtho[2,1-b]furan which comprises basically hydrolyzing the decalin derivative of claim 1.

3. A method according to claim 1 wherein the acid catalyst is a lower alkyl carboxylic acid, chloroacetic acid, dichloroacetic acid, trifluoroacetic acid, formic acid, phosphoric acid or a phosphate buffer.

4. A method according to claim 3 wherein the amount of acid used is from about 10 to about 5000 mole percent relative to the amount of sclareol oxide present.

5. A method according to claim 4 wherein the amount of acid is about 100 to about 4000 mole percent.

6. A method according to claim 4 wherein the amount of acid is about 3500 mole percent.

7. A method according to claim 1 wherein the solvent is tetrahydrofuran, dimethoxyethane, dioxane, ethyl ether, t-butanol, ethyl acetate, dichloromethane, dichloroethane, water, t-butyl methyl ether, toluene, hexane or a mixture thereof.

8. A method according to claim 1 wherein a temperature of about 0°C to about 50°C is used.

9. A method according to claim 1 wherein a temperature of from about 10°C to about 40°C is used.

10. A method according to claim 1 wherein the metallic halide reducing agent is a
ferrous halide, cuprous halide, chromium (II) halide, titanium (III) halide, vanadium (II) or (III) halide, cobalt (II) halide or a manganese (II) halide.

11. A decalin derivative of the formula 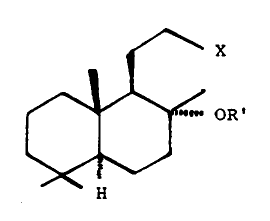 wherein X is chloro, bromo, or iodo and R' is an alkanoyl group of 2 to 5 carbons.

12. A sclareol oxide-13-hydroperoxide intermediate of the formula

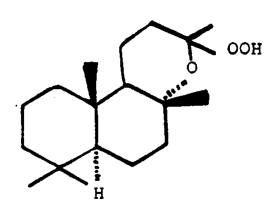

## Claims (Claims for the following Contracting State(s): ES)

1. A method for the preparation of a decalin derivative of the formula 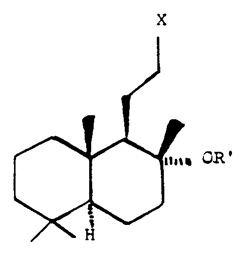 wherein X is chloro, bromo or iodo and R' is an alkanoyl group of 2 to 5 carbons, which comprises
treating sclareol oxide of the formula 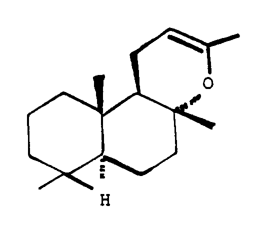 with hydrogen peroxide or a hydrogen peroxide generating system in the presence of an acid catalyst, and
treating the produced hydroperoxide intermediate with a metallic halide reducing agent, the halide being chloride, bromide or iodide.

2. A method for the production of dodecahydro-3a,6,6,9a-tetramethyl-naphtho[2,1-b]furan which comprises basically hydrolyzing the decalin derivative of claim 1.

3. A method according to claim 1 wherein the acid catalyst is a lower alkyl carboxylic acid, chloroacetic acid, dichloroacetic acid, trifluoroacetic acid, formic acid, phosphoric acid or a phosphate buffer.

4. A method according to claim 3 wherein the amount of acid used is from about 10 to about 5000 mole percent relative to the amount of sclareol oxide present.

5. A method according to claim 4 wherein the amount of acid is about 100 to about 4000 mole percent.

6. A method according to claim 4 wherein the amount of acid is about 3500 mole percent.

7. A method according to claim 1 wherein the solvent is tetrahydrofuran, dimethoxyethane, dioxane, ethyl ether, t-butanol, ethyl acetate, dichloromethane, dichloroethane, water, t-butyl methyl ether, toluene, hexane or a mixture thereof.

8. A method according to claim 1 wherein a temperature of about 0°C to about 50°C is used.

9. A method according to claim 1 wherein a temperature of from about 10°C to about 40°C is used.

10. A method according to claim 1 wherein the metallic halide reducing agent is a
ferrous halide, cuprous halide, chromium (II) halide, titanium (III) halide, vanadium (II) or (III) halide, cobalt (II) halide or a manganese (II) halide.

## Patentansprüche (Patentansprüche für folgende(n) Vertragsstaat(en): AT, BE, CH, DE, FR, GB, GR, IT, LI, LU, NL, SE)

1. Verfahren zur Herstellung eines Decalinderivats der Formel 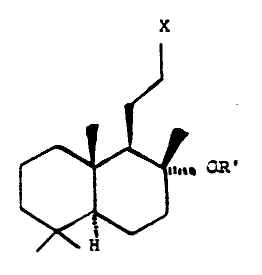 in der X ein Chlor-, Brom- oder Jodatom bedeutet und R' einen Alkanoylrest mit 2 bis 5 Kohlenstoffatomen bedeutet, umfassend
Behandeln von Sclareoloxid der Formel 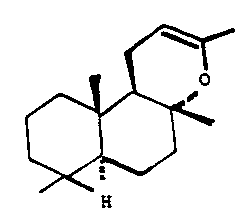 mit Wasserstoffperoxid oder einem Wasserstoffperoxid erzeugenden System in Gegenwart eines sauren Katalysators und
Behandeln des hergestellten Hydroperoxid-Zwischenprodukts mit einem Metallhalogenid-Reduktionsmittel, wobei Halogenid Chlorid, Bromid oder Jodid bedeutet.

2. Verfahren zur Herstellung von Dodecahydro-3a,6,6,9a-tetramethylnaphtho[2,1-b]furan, umfassend basische Hydrolyse des Decalinderivats von Anspruch 1.

3. Verfahren nach Anspruch 1, wobei der saure Katalysator eine Niederalkylcarbonsäure, Chloressigsäure, Dichloressigsäure, Trifluoressigsäure, Ameisensäure, Phosphorsäure oder ein Phosphatpuffer ist.

4. Verfahren nach Anspruch 3, wobei die verwendete Säuremenge etwa 10 bis etwa 5000 Mol-% bezogen auf die Menge des vorliegenden Sclareoloxids beträgt.

5. Verfahren nach Anspruch 4, wobei die Säuremenge etwa 100 bis etwa 4000 Mol-% beträgt.

6. Verfahren nach Anspruch 4, wobei die Säuremenge etwa 3500 Mol-% beträgt.

7. Verfahren nach Anspruch 1, wobei das Lösungsmittel Tetrahydrofuran, Dimethoxyethan, Dioxan, Diethylether, tert.-Butanol, Essigsäureethylester, Dichlormethan, Dichlorethan, Wasser, tert.-Butylmethylether, Toluol, Hexan oder ein Gemisch davon ist.

8. Verfahren nach Anspruch 1, wobei eine Temperatur von etwa 0°C bis etwa 50°C verwendet wird.

9. Verfahren nach Anspruch 1, wobei eine Temperatur von etwa 10°C bis etwa 40°C verwendet wird.

10. Verfahren nach Anspruch 1, wobei das Metallhalogenid-Reduktionsmittel ein Eisen(II)-halogenid, Kupfer(I)-halogenid, Chrom(II)-halogenid, Titan(III)-halogenid, Vanadin(II)- oder (III)-halogenid, Kobalt(II)-halogenid oder Mangan(II)-halogenid ist.

11. Decalinderivat der Formel 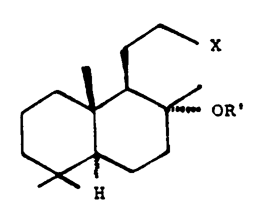 in der X ein Chlor-, Brom- oder Iodatom bedeutet und R' einen Alkanoylrest mit 2 bis 5 Kohlenstoffatomen darstellt.

12. Sclareoloxid-13-hydroperoxid-Zwischenverbindung der Formel

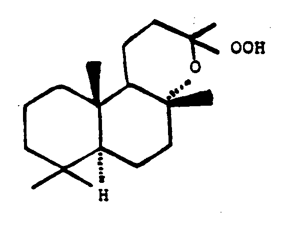

## Patentansprüche (Patentansprüche für folgende(n) Vertragsstaat(en): ES)

1. Verfahren zur Herstellung eines Decalinderivats der Formel 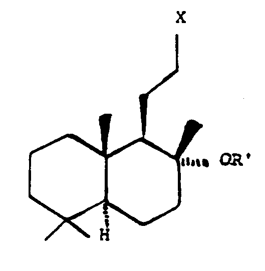 in der X ein Chlor-, Brom- oder Jodatom bedeutet und R' einen Alkanoylrest mit 2 bis 5 Kohlenstoffatomen bedeutet, umfassend
Behandeln von Sclareoloxid der Formel 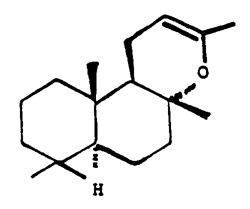 mit Wasserstoffperoxid oder einem Wasserstoffperoxid erzeugenden System in Gegenwart eines sauren Katalysators und
Behandeln des hergestellten Hydroperoxid-Zwischenprodukts mit einem Metallhalogenid-Reduktionsmittel, wobei Halogenid Chlorid, Bromid oder Jodid bedeutet.

2. Verfahren zur Herstellung von Dodecahydro-3a,6,6,9a-tetramethylnaphtho[2,1-b]furan, umfassend basische Hydrolyse des Decalinderivats von Anspruch 1.

3. Verfahren nach Anspruch 1, wobei der saure Katalysator eine Niederalkylcarbonsäure, Chloressigsäure, Dichloressigsäure, Trifluoressigsäure, Ameisensäure, Phosphorsäure oder ein Phosphatpuffer ist.

4. Verfahren nach Anspruch 3, wobei die verwendete Säuremenge etwa 10 bis etwa 5000 Mol-% bezogen auf die Menge des vorliegenden Sclareoloxids beträgt.

5. Verfahren nach Anspruch 4, wobei die Säuremenge etwa 100 bis etwa 4000 Mol-% beträgt.

6. Verfahren nach Anspruch 4, wobei die Säuremenge etwa 3500 Mol-% beträgt.

7. Verfahren nach Anspruch 1, wobei das Lösungsmittel Tetrahydrofuran, Dimethoxyethan, Dioxan, Diethylether, tert.-Butanol, Essigsäureethylester, Dichlormethan, Dichlorethan, Wasser, tert.-Butylmethylether, Toluol, Hexan oder ein Gemisch davon ist.

8. Verfahren nach Anspruch 1, wobei eine Temperatur von etwa 0°C bis etwa 50°C verwendet wird.

9. Verfahren nach Anspruch 1, wobei eine Temperatur von etwa 10°C bis etwa 40°C verwendet wird.

10. Verfahren nach Anspruch 1, wobei das Metallhalogenid-Reduktionsmittel ein Eisen(II)-halogenid, Kupfer(I)-halogenid, Chrom(II)-halogenid, Titan(III)-halogenid, Vanadin(II)- oder (III)-halogenid, Kobalt(II)-halogenid oder Mangan(II)-halogenid ist.

## Revendications (Revendications pour l'(les) Etat(s) contractant(s) suivant(s): AT, BE, CH, DE, FR, GB, GR, IT, LI, LU, NL, SE)

1. Procédé pour la préparation d'un dérivé de décaline de formule 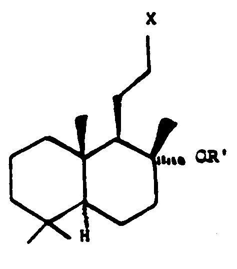 dans laquelle X est un groupe chloro, bromo ou iodo et R' est un groupe alcanoyle de 2 à 5 atomes de carbone, qui comprend les étapes consistant
à traiter l'oxyde de sclaréol de formule 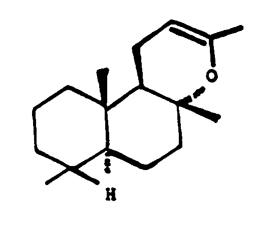 avec du peroxyde d'hydrogène ou un système engendrant du peroxyde d'hydrogène en présence d'un catalyseur acide, et
à traiter l'intermédiaire hydroperoxyde produit avec un agent de réduction halogénure métallique, l'halogénure étant le chlorure, le bromure ou l'iodure.

2. Procédé pour la production de dodécahydro-3a,6,6,9a-tétraméthyl-naphto[2,1-b]furanne qui consiste à hydrolyser par voie basique le dérivé de décaline selon la revendication 1.

3. Procédé selon la revendication 1, dans lequel le catalyseur acide est un acide alkyl(inférieur)carboxylique, l'acide chloroacétique, l'acide dichloroacétique, l'acide trifluoroacétique, l'acide formique, l'acide phosphorique ou un tampon phosphate.

4. Procédé selon la revendication 3, dans lequel la quantité d'acide utilisée est d'environ 10 à environ 5 000 pourcent en moles par rapport à la quantité d'oxyde de sclaréol présent.

5. Procédé selon la revendication 4, dans lequel la quantité d'acide est d'environ 100 à environ 4 000 pourcent en moles.

6. Procédé selon la revendication 4, dans lequel la quantité d'acide est d'environ 3 500 pourcent en moles.

7. Procédé selon la revendication 1, dans lequel le solvant est le tétrahydrofuranne, le diméthoxyéthane, le dioxanne, l'éther éthylique, le t-butanol, l'acétate d'éthyle, le dichlorométhane, le dichloroéthane, l'eau, l'éther méthylique de t-butyle, le toluène, l'hexane ou un de leurs mélanges.

8. Procédé selon la revendication 1, dans lequel on utilise une température d'environ 0°C à environ 50°C.

9. Procédé selon la revendication 1, dans lequel on utilise une température d'environ 10°C à environ 40°C.

10. Procédé selon la revendication 1, dans lequel l'agent de réduction halogénure métallique est un halogénure ferreux, un halogénure cuivreux, un halogénure de chrome (II), un halogénure de titane (III), un halogénure de vanadium (II) ou (III), un halogénure de cobalt (II) ou un halogénure de manganèse (II).

11. Dérivé de décaline de formule 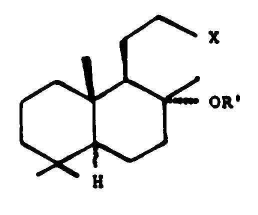 dans laquelle X est un groupe chloro, bromo, ou iodo et R' est un groupe alcanoyle de 2 à 5 atomes de carbone.

12. Intermédiaire oxyde-13-hydroperoxyde de sclaréol de formule

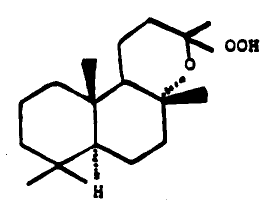

## Revendications (Revendications pour l'(les) Etat(s) contractant(s) suivant(s): ES)

1. Procédé pour la préparation d'un dérivé de décaline de formule 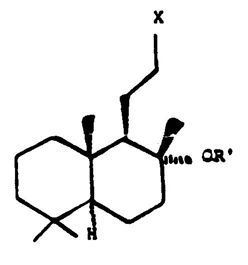 dans laquelle X est un groupe chloro, bromo ou iodo et R' est un groupe alcanoyle de 2 à 5 atomes de carbone, qui comprend les étapes consistant
à traiter l'oxyde de sclaréol de formule 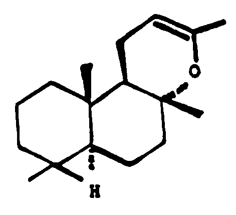 avec du peroxyde d'hydrogène ou un système engendrant du peroxyde d'hydrogène en présence d'un catalyseur acide, et
à traiter l'intermédiaire hydroperoxyde produit avec un agent de réduction halogénure métallique, l'halogénure étant le chlorure, le bromure ou l'iodure.

2. Procédé pour la production de dodécahydro-3a,6,6,9a-tétraméthyl-naphto[2,1-b]furanne qui consiste à hydrolyser par voie basique le dérivé de décaline selon la revendication 1.

3. Procédé selon la revendication 1, dans lequel le catalyseur acide est un acide alkyl(inférieur)carboxylique, l'acide chloroacétique, l'acide dichloroacétique, l'acide trifluoroacétique, l'acide formique, l'acide phosphorique ou un tampon phosphate.

4. Procédé selon la revendication 3, dans lequel la quantité d'acide utilisée est d'environ 10 à environ 5 000 pourcent en moles par rapport à la quantité d'oxyde de sclaréol présent.

5. Procédé selon la revendication 4, dans lequel la quantité d'acide est d'environ 100 à environ 4 000 pourcent en moles.

6. Procédé selon la revendication 4, dans lequel la quantité d'acide est d'environ 3 500 pourcent en moles.

7. Procédé selon la revendication 1, dans lequel le solvant est le tétrahydrofuranne, le diméthoxyéthane, le dioxanne, l'éther éthylique, le t-butanol, l'acétate d'éthyle, le dichlorométhane, le dichloroéthane, l'eau, l'éther méthylique de t-butyle, le toluène, l'hexane ou un de leurs mélanges.

8. Procédé selon la revendication 1, dans lequel on utilise une température d'environ 0°C à environ 50°C.

9. Procédé selon la revendication 1, dans lequel on utilise une température d'environ 10°C à environ 40°C.

10. Procédé selon la revendication 1, dans lequel l'agent de réduction halogénure métallique est un halogénure ferreux, un halogénure cuivreux, un halogénure de chrome (II), un halogénure de titane (III), un halogénure de vanadium (II) ou (III), un halogénure de cobalt (II) ou un halogénure de manganèse (II).
